Europäisches Patentamt

European Patent Office

Office européen des brevets

(11) Veröffentlichungsnummer: **0 359 987**

**A2**

## (12) EUROPÄISCHE PATENTANMELDUNG

(21) Anmeldenummer: **89114964.3**

(51) Int. Cl.⁵ **A61B 17/34**

(22) Anmeldetag: **12.08.89**

(30) Priorität: **20.09.88 DE 8811891 U**

(43) Veröffentlichungstag der Anmeldung:
**28.03.90 Patentblatt 90/13**

(84) Benannte Vertragsstaaten:
**AT BE CH DE ES FR GB GR IT LI LU NL SE**

(71) Anmelder: **B. Braun Melsungen AG
Carl-Braun Strasse
D-3508 Melsungen(DE)**

(72) Erfinder: **Haindl, Hans-Günter, Dr.
Schöne Aussicht 4
D-4508 Melsungen(DE)**

(74) Vertreter: **Selting, Günther, Dipl.-Ing. et al
Patentanwälte von Kreisler, Selting, Werner
Deichmannhaus am Hauptbahnhof
D-5000 Köln 1(DE)**

(54) Stahlkanüle für die Spinal- und Periduralanästhesie.

(57) Um eine Stahlkanüle (10) für die Spinal- und Periduralanästhesie so zu optimieren, daß sie die Vorzüge eines dünnen Rohres mit verbesserter Spitzen-Lokalisierung durch erleichterte Überprüfung des Liquorrückflusses verbindet, ist erfindungsgemäß vorgesehen, daß der Außendurchmesser des zylindrischen Rohres (11) und der Durchmesser des Rohrkanals (15) auf einem vorderen zylindrischen Endabschnitt (11a;15a) verringert sind und daß ein in dem Rohrkanal (15) steckender Mandrin (30) den verengten Rohrkanal-Endabschnitt (15a) umfangsmäßig im wesentlichen ausfüllt.

EP 0 359 987 A2

FIG.1

## Stahlkanüle für die Spinal- und Periduralanästhesie

Die Erfindung betrifft eine Stahlkanüle für die Spinal-und Periduralanästhesie nach dem Oberbegriff des Anspruches 1.

Werden in der Spinal- oder Periduralanästhesie Kanülen mit schneidender oder reißender Einstechspitze benutzt, so besteht die Gefahr von Gefäß-. Dura- und Nervenverletzungen. Insbesondere können Blutungen im Periduralraum, vorübergehende bis persistierende neuronale Ausfälle und postspinaler Kopfschmerz auftreten. Der postspinale Kopfschmerz ist ein schwer behandelbarer Schmerz, der bis zu mehreren Tagen nach einer Spinalanästhesie bestehen kann und für den Patienten sehr quälend ist. Als Ursache hierfür wird heute das Liquorleck-Syndrom postuliert. Bei der Gestaltung von Spinalkanülen versucht man daher die Verletzung der Dura so gering wie möglich zu halten, damit nach abgeschlossener Punktion möglichst wenig Liquor aus der Dura aus treten kann. Außerdem wird versucht, den Liquorverlust durch Flachlagerung des Patienten klein zu halten.

Zur Kleinhaltung des Punktionsloches wurde das Rohr der Stahlkanüle insgesamt extrem dünn gestaltet und an der Spitze mit Quinkeschliff versehen. Bei solchen dünnen Spinalkanülen ist praktisch kein freier Liquorrückfluß mehr zu erzielen, was unerwünscht ist, weil das Erreichen des Zielortes mit der Spitze der Kanüle, das durch Liquorrückfluß angezeigt wird, nicht ausreichend präzise erkennbar ist. Ferner besteht bei sehr dünnen Spinalkanülen die Neigung zum Abknicken, so daß sie die harte Dura des Spinalkanals nicht geradlinig perforieren, sondern seitlich ausweichen und die vordere Öffnung des Rohrkanals, durch die Anästhetikum injiziert werden soll, den Spinalkanal nicht erreicht. Je dünner die Kanüle desto stärker gleitet ihre Einstechspitze auf der Schliffschrägen des Quinkeschliffes ab; die Stahlkanüle kann also aus mehreren Gründen einen anderen Weg nehmen, als der Anwender vorgesehen hat, und dieser Nachteil ist auch bei Verwendung von Führungskanülen nicht ganz zu vermeiden. Ferner ist bei sehr dünnen Spinalkanülen der Mandrin so dünn, daß sein Wiedereinschieben in die Stahlkanüle nach Kontrolle des Liquorrückflusses Schwierigkeiten durch das Erfordernis hoher Zielgenauigkeit bereitet. Hygienische Risiken und Infektionsgefahr für den Anwender durch Stichverletzungen sind nicht ausgeschlossen.

Zur möglichst verletzungsfreien Durchdringung der Dura mit einer Spinalkanüle sind ferner sogenannte atraumatische Spitzen an Spinalkanülen ausgebildet worden. Diese haben die Form eines geschlossenen spitzen Kegels, hinter dem in der Rohrwand eine in den Rohr kanal führende Öffnung ausgebildet ist (DE-PS 30 20 926). Derartige atraumatische Spitzen haben erheblich höhere Penetrationskräfte als schneidende Spitzen und sie erzeugen beim Einstich in die Dura nur ein kleines Punktionsloch, das überwiegend durch Verdrängung der elastischen Fasern der Dura erzeugt wird und sich nach Herausziehen der Kanüle weitgehend schließt. Auch bei solchen Spinalkanülen muß allerdings der Mandrin zum Überprüfen des Liquorrückflusses vollständig herausgezogen werden und das Wiedereinführen ist problematisch. Außerdem lassen die atraumatischen Spinalkanülen sich nicht in so dünnen Größen herstellen, wie die zuerst erwähnten Quinkekanülen, so daß eine für die Punktion der Dura an sich günstige Kombination eines sehr dünnen Rohres mit atraumatischer Spitze nicht möglich ist.

Zur Verbesserung des Durchtritts einer stabilen Stahlkanüle mit scharfem Schräganschliff durch die Haut eines Patienten ist gemäß US-PS 4 335 718 der Außendurchmesser des Rohres am äußersten vorderen Ende verringert worden. Der Durchmesser des Rohrkanals ist von einem zum anderen Ende gleichbleibend oder am äußersten vorderen Ende vergrößert ausgebildet. Die zweite Gestaltung ist zur Punktion mit einem Mandrin nicht brauchbar, weil er den vergrößerten Querschnitt des vorderen Endes des Rohrkanals nicht ausfüllen kann und auch die erste Gestaltung ist - abgesehen von dem unzweckmäßigen Schräganschliff - für die Spinal- und Periduralanästhesie nicht out geeignet, weil sie keine günstige Möglichkeit der Überprüfung des freien Liquorrückflusses bietet.

Der Erfindung liegt die Aufgabe zugrunde, eine Stahlkanüle für die Spinal- und Periduralanästhesie der eingangs erwähnten Art so zu optimieren, daß sie die Vorzüge eines dünnen Rohres mit verbesserter Spitzen-Lokalisierung durch erleichterte Überprüfung des Liquorrückflusses verbindet.

Diese Aufgabe wird erfindungsgemäß dadurch gelöst, daß der Außendurchmesser des Rohres und der Durchmesser des Rohrkanals auf einem vorderen zylindrischen Endabschnitt verringert sind und daß der Mandrin den verengten Rohrkanal-Endabschnitt umfangsmäßig im wesentlichen ausfüllt.

Eine in dieser Weise ausgebildete Stahlkanüle ist nur in dem Bereich dünn, in dem sie die Dura perforiert. In ihrem restlichen, längeren Verlauf ist sowohl ihr Außendurchmesser als auch ihr Innendurchmesser, d.h. der Querschnitt ihres Rohrkanals, größer. Durch diese unterschiedliche Bemessung von zwei koaxial aufeinanderfolgenden Abschnitten des Rohres erhält die mit dem dünneren Endabschnitt leichter und mit sehr kleinem Punktionsloch durch die harte Hirnhaut (Dura) des Spinalkanals einführbare Stahlkanüle gute Stabilität und in dem vergrößerten Rohrkanalquerschnitt wird freier Liquorrückfluß erzielt. Dabei ist hinsichtlich der Handhabung und der Hygiene besonders vorteilhaft, daß zum Überprüfen der Position der Rohrspitze durch Kontrolle des Liquorrückflusses der Mandrin nicht mehr vollständig aus dem Rohr der Stahlkanüle herausgezogen werden muß. Ein Zurückziehen des Mandrins um die Länge des verengten Rohrkanal-Endabschnittes, der ca. 15 mm lang ist, verbindet die Öffnung des Rohrkanals mit seinem Abschnitt größeren Durchmessers und gewähr leistet einen hinreichenden Liquorrückfluß. Der Liquorrückfluß erfolgt erheblich leichter als bei einer durchgängig dünnen Kanüle, da der Durchflußwiderstand in dem Rohrkanal größeren Durchmessers erheblich geringer ist.

Zur Herstellung einer solchen Stahlkanüle ist erfindungsgemäß vorgesehen, daß das Rohr an seinem vorderen Endabschnitt auf einem Dorn auf geringere Stärke zusammengehämmert wird und daß Wandverdickungen übergeschliffen werden. Beispielsweise bei einem Rohr von 22 G Stärke (0,71 mm) wird der vordere Endabschnitt auf eine Stärke von ca. 26 G (0,45 mm) zusammengehämmert. An diesem dünnen vorderen Endabschnitt wird dann eine Einstechspitze angebracht, die nur geringe seitliche Versatzkräfte aufweisen darf, damit die zu durchdringenden Gewebefasern nur auseinandergeschoben, möglichst aber nicht zerschnitten werden. Als Einstechspitze geeignet ist erfindungsgemäß eine Ausbildung gemäß Anspruch 4. Eine solche Einstechspitze mit seitwärts gerichteter Öffnung zeichnet sich durch verringerten Stanzeffekt aus, weil von dem lancettförmigen, einseitig gekrümmten vorderen Einstechteil aus eine symmetrische Aufweitung des Gewebes bis zum Außendurchmesser des Rohres der Stahlkanüle erfolgt, die sich nach Herausziehen der Stahlkanüle dicht verschließt.

Auch eine Einstechspitze in Form eines geschlossenen spitzen Kegels, hinter dem in der Rohrwand die Öffnung ausgebildet ist, eignet sich für den dünnen vorderen Endabschnitt der erfindungsgemäßen Stahlkanüle für die Spinal- und Periduralanästhesie.

Vorteilhafterweise ist der Übergang vom größeren zum geringeren Durchmesser des Rohres und des Rohrkanals als langgestreckter konischer Abschnitt gestaltet, der keine Stufe bildet, sondern einen kontinuierlichen Übergang zwischen den beiden koaxialen Durchmessern vermittelt. Beim Außendurchmesser des Rohres ergibt sich hierdurch eine Verhinderung plötzlicher Gewebeaufweitung und beim Innendurchmesser (Rohrkanal) hat der langgestreckte konische Abschnitt die Aufgabe, die Spitze des Mandrins gleichmäßig in den verengten Rohrkanal-Endabschnitt zu leiten, wenn zur Kontrolle des Liquorrückflusses der Mandrin in regelmäßigen Abständen um ein kurzes Stück in den Rohrkanal größeren Querschnitts zurückgezogen worden ist.

Wenn der Mandrin aus dem Rohr vollständig herausgezogen wurde und sein erneutes Einstecken erwünscht ist, wird dies dadurch erleichtert, daß das Rohr an einer transparenten Griffhülse mit konisch nach hinten erweitertem Hohlraum befestigt ist. Die konische Erweiterung bildet eine Art Trichter, die das Einstecken des dünnen Mandrins ohne besondere Zielgenauigkeit vereinfacht.

In der Zeichnung ist ein Ausführungsbeispiel der Erfindung schematisch dargestellt.

Es zeigen:

Fig. 1 einen Längsschnitt durch eine Stahlkanüle mit einer Ausführung der Einstechspitze mit eingesetztem Mandrin und

Fig. 2 einen Längsschnitt durch eine Stahlkanüle mit abgewandelter Einstechspitze.

Die Stahlkanüle 10 für die Spinal- und Periduralanästhesie besteht im wesentlichen aus einem geraden zylindrischen Rohr 11 aus Stahl, an dessen vorderem Ende eine Einstechspitze 12 ausgebildet ist und deren hinteres Ende an einer Griffhülse 13 aus transparentem Material befestigt ist. In dem Rohr 11 ist ein zylindrischer Rohrkanal 15 vorhanden, der vorne an einer seitwärts gerichteten Öffnung 14 und hinten an einem zentralen Durchlaß 16 mündet. Zur Befestigung des hinteren Endes des Rohres 11 an der Griffhülse 13 dient ein zylindrischer Stutzen 20, in den das Rohrende eingeklebt ist. Die Griffhülse 13 ist konzentrisch von einem konisch nach hinten erweiterten Hohlraum 21 durchsetzt, der aus zwei koaxial aufeinanderfolgenden konischen Abschnitten 22,23 unterschiedlicher Öffnungsweite zusammengesetzt ist. Der hintere konische Abschnitt 23 endet offen und an seinem Rand ist die Griffhülse 13 mit radial nach außen gerichteten Ansätzen 25 versehen, die als Verriegelungselemente für das Kupplungsteil eines nicht gezeichneten Spritze dienen.

Der Außendurchmesser eines vorderen kurzen, zylindrischen Endabschnittes 11a des Rohres 11 ist geringer als der Außendurchmesser eines langen, zylindrischen Rohrabschnittes 11b und auch der Innendurchmesser des Rohres 11, d.h. der Querschnitt seines Rohrkanals 15, verändert sich von einem langen Rohrkanalabschnitt 15b größeren Durchmessers zu einem kurzen Rohrkanal-Endabschnitt 15a kleineren Durchmessers. Das Rohr 11 hat am vorderen Ende seines Rohrabschnittes 11b größeren Durchmessers einen langgestreckten konischen Abschnitt 11c, der einen allmählichen Übergang sowohl des äußeren als auch des inneren Durchmessers des Rohres 11 auf den verengten Endabschnitt 11a,15a bewirkt.

In dem Rohr 11 und der Griffhülse 13 steckt konzentrisch ein Mandrin 30 aus Stahl. Der Mandrin 30 hat kreisförmigen Querschnitt und seine Dicke ist so be messen, daß er den verengten Rohrkanal-Endabschnitt 15a umfangsmäßig annähernd ausfüllt. Die Spitze 31 des Mandrins 30 ist so geschliffen, daß sie das Schliffauge 17 der Öffnung 14 absatzfrei ausfüllt. Am anderen Ende des Mandrins 30 ist ein üblicher Ansatz 26 befestigt, der mit einem Außenkegel 27 in den Innenkegel des Hohlraumabschnittes 23 abdichtend passend eingreift und mit einem im Abstand zu dem Außenkegel 27 angebrachten Umfangsmantel 28 die radialen Ansätze 25 kappenartig umfängt.

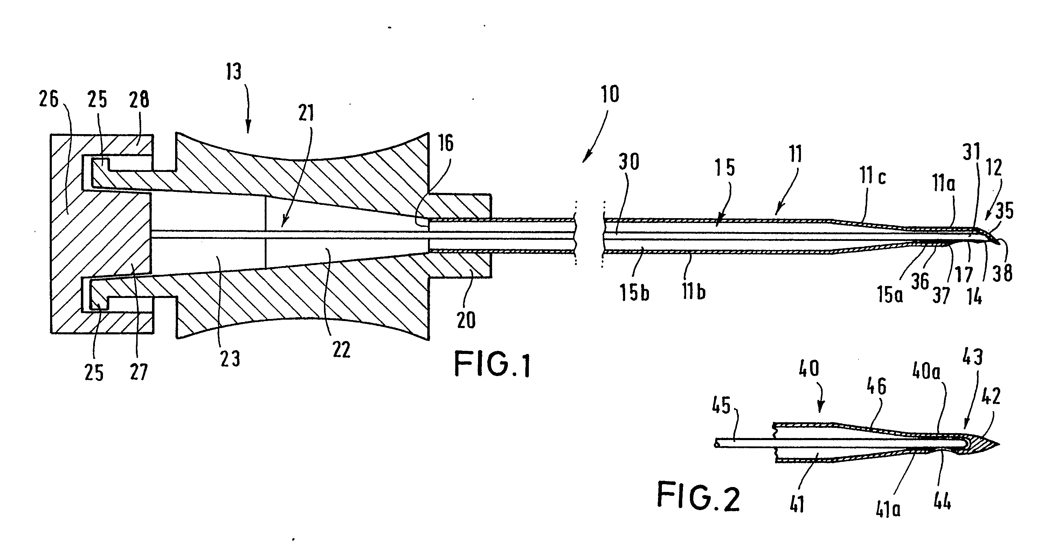

Die Einstechspitze 12 weist bei dem Beispiel der Fig. 1 eine konvex gekrümmte Wandseite 35 auf, die mit der gegenüberliegenden geraden Wandseite 36 die seitliche Öffnung 14 begrenzt, welche mit einer hinteren Schneide 37 und einem in einer Spitze endenden lancettförmigen vorderen Einstechteil 38 versehen ist. Die Spitze des Einstechteils 38 liegt in dem Bereich zwischen zwei gedachten parallelen Linien, die die Innenfläche und die Außenfläche der geraden Wandseite 36 über die hintere Schneide 37 hinaus nach vorne verlängern. Der lancettförmige vordere Einstechteil 38 ist mit Facettenschliff versehen und bildet mit einem ergänzenden Öffnungs-Randschliff das Schliffauge 17, das von der angepaßten Schlifffläche der Spitze 31 des Mandrins 30 passend verschlossen wird. Die dargestellte Einstechspitze 12 zeichnet sich durch verringerten Stanzeffekt aus, der in Verbindung mit dem verengten vorderen Endabschnitt 11a des Rohres 11 dazu führt, daß die Dura praktisch nicht verletzt wird und die Punktionsöffnung sich nach Herausziehen der Kanüle sofort wieder schließt, so daß nach abgeschlossener Punktion nur sehr wenig Liquor aus der Dura austreten kann und postspinalem Kopfschmerz entgegengewirkt wird.

Freier Liquorrückfluß zur Lokalisierung des Einstechteiles 12 ist durch den Rohrkanalabschnitt 15b größeren Durchmessers gewährleistet, sobald der Mandrin 30 um die Länge des verengten Rohrkanal-Endabschnittes 15a zurückgezogen ist.

Während des Vorschubes der Stahlkanüle 10 in Richtung auf die Dura wird in regelmäßigen Abständen kontrolliert, ob der Spinalraum bereits erreicht ist. Diese Kontrolle ergibt sich durch Rückfluß des Liquors. Zu seiner Freigabe wird die Griffhülse 13 mit dem Mandrin 30 soweit zurückgezogen, bis die Spitze 31 des Mandrins 30 den verengten Rohrkanal-Endabschnitt 15a verlassen hat. Da der vordere Endabschnitt 11a vorzugsweise etwa 15 mm lang ist, muß der Anwender den Mandrin nur ca. 15 mm zurückziehen, um den Rückfluß des Liquors beurteilen zu können, der infolge des verringerten Durchflußwiderstandes in dem Rohrkanalabschnitt 15b unbehindert ist.

Bei dem Beispiel der Fig. 2 ist ein zylindrisches gerades Rohr 40 an seinem vorderen Endabschnitt 40a dünner ausgebildet und entsprechend ist auch der vordere zylindrische Rohrkanal-Endabschnitt 41a des Rohrkanals 41 verengt. Der vordere Endabschnitt 40a ist gerade und er ist von einer als geschlossener spitzer Kegel 42 gestalteten Einstechspitze 43 abgeschlossen. Unmittelbar hinter dem Kegel 42 befindet sich in der Rohrwand eine Öffnung 44 mit kreisförmigen oder quadratischem Querschnitt, deren Rand einen Spezialschliff aufweist. Auch in dieser Stahlkanüle steckt ein Mandrin 45, der während der Punktion die Öffnung 44 verschließt und dessen Zurückziehung aus dem vorderen Endabschnitt 40a geringeren Außen- und Innendurchmessers des Rohres 40 freien Liquorrückfluß ermöglicht, der anzeigt, daß der Spinalraum erreicht ist. Der Übergang zwischen dem Rohrabschnitt 40 größeren Durchmessers und dem Endabschnitt 40a verringerten Durchmessers erfolgt auch bei diesem Beispiel über einen langgestreckten konischen Abschnitt 46, der die Einführung des Mandrins 45 in den verengten Rohrkanal-Endabschnitt 41a erleichtert und einen anschlagfreien Einstich des dünnen Endabschnittes 40a in die harte Hirnhaut des Spinalkanals ermöglicht.

## Ansprüche

1. Stahlkanüle (10) für die Spinal- und Periduralanästhesie, bestehend aus einem geraden zylindrischen Rohr (11) mit einem Rohrkanal (15), in dem ein herausziehbarer Mandrin (30) steckt, mit einer Einstechspitze (12) und mit einer vorderen Öffnung (14) des Rohrkanals (15),
**dadurch gekennzeichnet,**
daß der Außendurchmesser des Rohres (11) und der Durchmesser des Rohrkanals (15) auf einem vorderen zylindrischen Endabschnitt (11a;15a) verringert sind und daß der Mandrin (30) den verengten Rohrkanal-Endabschnitt (15a) umfangsmäßig im wesentlichen ausfüllt.

2. Stahlkanüle nach Anspruch 1, dadurch gekennzeichnet, daß der Übergang vom größeren zum geringeren Durchmesser des Rohres (11) und des Rohrkanals (15) als langgestreckter konischer Abschnitt (11c) ausgebildet ist.

3. Stahlkanüle nach Anspruch 1 oder 2, dadurch gekennzeichnet, daß das Rohr (11) an einer transparenten Griffhülse (13) mit konisch nach hinten erweitertem Hohlraum (21) befestigt ist.

4. Stahlkanüle nach einem der Ansprüche 1 bis 3, dadurch gekennzeichnet, daß die Einstechspitze (12) des vorderen Abschnittes (11a) eine konvex gekrümmte Wandseite (35) aufweist, die mit der gegenüberliegenden geraden Wandseite (36) eine seitliche Öffnung (14) begrenzt, welche mit einer hinteren Schneide (37) und einem lancettförmigen vorderen Einstechteil (38) versehen ist, dessen Spitze in dem Bereich zwischen zwei gedachten parallelen Linien liegt, die die Innenfläche und die Außenfläche der geraden Wandseite (36) über die hintere Schneide (37) hinaus nach vorne verlängern.

5. Stahlkanüle nach Anspruch 4, dadurch gekennzeichnet, daß der Einstechteil (12) mit Facettenschliff versehen ist.

6. Stahlkanüle nach einem der Ansprüche 1 bis 3, dadurch gekennzeichnet, daß die Einstechspitze (43) des vorderen Endabschnittes (40a) als geschlossener spitzer Kegel (42) gestaltet ist und daß die Öffnung (44) hinter dem Kegel (42) in der Wand des Rohres (40) ausgebildet ist.

7. Verfahren zur Herstellung einer Stahlkanüle nach einem der Ansprüche 1 bis 6, dadurch gekennzeichnet, daß das Rohr an seinem vorderen Endabschnitt auf einem Dorn auf geringere Stärke zusammengehämmert wird, daß an dem vorderen Endabschnitt eine Einstechspitze und eine Öffnung mit Anschliff angebracht werden und daß Wandverdickungen übergeschliffen werden.

FIG.1

FIG.2